(19) Europäisches Patentamt
European Patent Office
Office européen des brevets

(11) EP 0 684 230 B1

(12) **EUROPEAN PATENT SPECIFICATION**

(45) Date of publication and mention of the grant of the patent:
**03.07.2002 Bulletin 2002/27**

(51) Int Cl.[7]: **C07D 205/08**, C07F 7/18, C07D 487/04

(21) Application number: **95111576.5**

(22) Date of filing: **11.03.1994**

(54) **4- (R)-1'-formylethyl azetidin-2-one derivatives**

4-(R)-1'-Formylethyl Azetidin-2-on-Derivate

Dérivés de 4-(R)-1'-formyléthyl-azetidin-2-one

(84) Designated Contracting States:
**CH DE FR GB IT LI NL**

(30) Priority: **12.03.1993 JP 7748493**

(43) Date of publication of application:
**29.11.1995 Bulletin 1995/48**

(62) Document number(s) of the earlier application(s) in accordance with Art. 76 EPC:
**94301775.6 / 0 614 903**

(73) Proprietor: **Takasago International Corporation Tokyo (JP)**

(72) Inventors:
- **Saito, Takao c/o Takasago Intern. Corp. Fine Chem. Hiratsuka-shi Kanagawa (JP)**
- **Matsumura, Kazuhiko c/o Takasago Intern. Corp. Hiratsuka-shi Kanagawa (JP)**
- **Kato, Yasushi c/o Takasago Intern. Corp. Hiratsuka-shi Kanagawa (JP)**
- **Sayo, Noboru c/o Takasago Intern. Corporation Hiratsuka-shi Kanagawa (JP)**
- **Kumobayashi, Hidenori c/o Takasago Intern. Corp. Hiratsuka-shi Kanagawa (JP)**

(74) Representative: **Dixon, Donald Cossar et al
Wilson Gunn Gee
Chancery House
Chancery Lane
London WC2A 1QU (GB)**

(56) References cited:

**EP-A- 0 010 317**
**EP-A- 0 089 139**
**EP-A- 0 113 101**
**EP-A- 0 180 189**
**EP-A- 0 188 816**
**WO-A-88/01619**

- **SASAKI ET AL: 'Synthetic Studies of Carbapenem and Penem Antibiotics. III. A Synthesis of a Key Intermediate for 1-beta-Methylcarbapenem' CHEM. PHARM. BULL. vol. 40, no. 5, 1992, pages 1098 - 1104**
- **SHIRAI ET AL: 'A New Synthetic Route to the Key Precursor of 1-beta-Methylcarbapenem Antibiotics from (S)-Methyl 3-Hydroxy-2-methylpropanoate' CHEMISTRY LETTERS 1989, pages 445 - 448**
- **SHIRAI ET AL: 'A novel, double asymmetric aldol approach to the synthesis of a 1-beta-methyl carbapenem antibiotic precursor' TETRAHEDRON LETTERS vol. 29, no. 49, 1988, pages 6461 - 6464**
- **KAWABATA ET AL: 'A novel and efficient synthesis of the key intermediate of 1-beta-methylcarbapenem antibiotics employing [2+2]-cycloaddition reaction of diketene with a chiral imine' TETRAHEDRON vol. 44, no. 8, 1988, pages 2149 - 2165**
- **KAWABATA ET AL: 'A novel and efficient synthesis of the key intermediate of 1-beta-methylcarbapenem antibiotics from (S)-methyl 3-hydroxy-2-methylpropionate' TETRAHEDRON LETTERS vol. 27, no. 51, 1986, pages 6241 - 6244**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- SHIH ET AL: 'Synthetic carbapenem antibiotics. III. 1-Methyl thienamycin' TETRAHEDRON LETTERS vol. 26, no. 5, 1985, pages 587 - 590
- BERKS, ANDREW H: 'TETRAHEDRON REPORT NUMBER 387. Preparations of Two Pivotal Intermediates for the Synthesis of 1-beta-Methyl Carbapenem Antibiotics' TETRAHEDRON vol. 52, no. 2, 1996, pages 331 - 375

## Description

This invention relates to 4-[(R)-1'-formylethyl]azetidin-2-one derivatives and, more particularly, to such derivatives serving as useful intermediates for the preparation of carbapenem antibiotics. Such derivatives are producable by olefin hydroformylation using phosphine compounds in the form of their transition metal complexes, or in the form of compounds with transition metals.

A large number of reports have been made on transition metal complex catalysts useful for organic syntheses, such as asymmetric hydrogenation, asymmetric hydrosilylation, and asymmetric hydroformylation.

In particular, many complexes exhibiting excellent catalytic activity in asymmetric syntheses are found among those comprising a transition metal, e.g., rhodium or ruthenium, and an optically active tertiary phosphine compound as a ligand. To improve the activity of these catalysts, various phosphine compounds having a special structure have been developed to date as disclosed, e.g., in Nippon Kagakukai (ed.), KAGAKU SOSETSU, Vol. 32, "YUKI KINZOKU NO KAGAKU", pp. 237-238 (1982).

Attention being directed only to asymmetric hydroformylation using a transition metal-phosphine complex as a catalyst, known techniques include a reaction using a rhodium complex containing optically active 2,3-O-isopropylidene-2,3-dihydroxy-1,4-bis(diphenylphosphino)butane (hereinafter abbreviated as DIOP) as a ligand (see J. Org. Chem., Vol. 46, p. 4422 (1981)), a reaction using a rhodium complex containing an optically active bidentate phosphine compound (e.g., DIOP) as a ligand (see Bull. Chem. Soc. Jpn., Vol. 52, p. 2605 (1979)), and asymmetric hydroformylation of methyl acetamidoacrylate using a rhodium complex containing DIOP, etc. as a ligand (see Tetrahedron Asymmetry, Vol. 10, p. 693 (1990)).

Known complexes containing an optically active tertiary phosphite as a ligand include those containing a bis (triarylphosphite) ligand having an optically active binaphthyl skeleton disclosed in Tetrahedron Asymmetry, Vol. 3, p. 583 (1992). Asymmetric hydroformylation of vinyl acetate using a rhodium complex containing this ligand has been reported.

On the other hand, carbapenem antibiotics have recently undergone marked development, and preparation of 4-[(R)-1'-carboxyethyl]azetidin-2-one derivatives represented by formula (VIII):

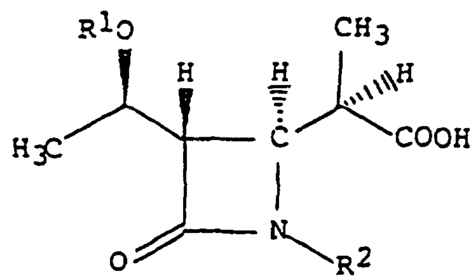

(VIII)

wherein $R^1$ represents a hydrogen atom or a hydroxyl-protecting group; and $R^2$ represents a hydrogen atom or an amino-protecting group, which are important intermediates for carbapenem antibiotics have been extensively studied.

The carbapenem antibiotics having no substituent at the 1-position of the carbapenem skeleton are chemically unstable at high concentrations and are readily metabolized by renal dehydropeptidase. It is known that introduction of an alkyl group in β-configuration into the 1-position provides a compound which has increased stability against renal dehydropeptidase and therefore can be used alone without combined use of a renal dehydropeptidase inhibitor.

A number of reports have been made on synthesis of the compound of formula (VIII). In particular, processes comprising nucleophilic substitution to the 4-position of a 4-acetoxyazetidin-2-one derivative represented by formula (IX):

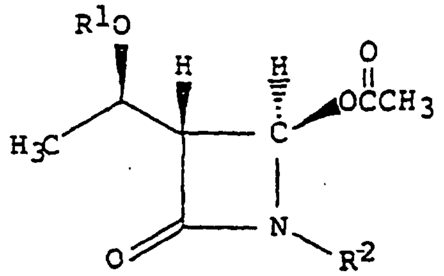

(IX)

wherein $R^1$ and $R^2$ are as defined above, by means of various nucleophiles are known.

Other known processes for synthesizing the intermediate compound (VIII) include a process comprising alkylation of a compound represented by formula (X):

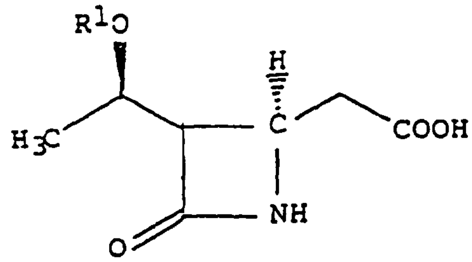

(X)

wherein $R^1$ is as defined above, with lithium diisopropylamide and a process comprising catalytic hydrogenation of the exo-methylene group of a compound represented by formula (XI):

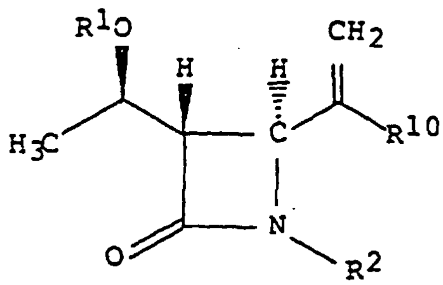

(XI)

wherein $R^1$ and $R^2$ are as defined above; and $R^{10}$ represents an alkyl group, a carboxyl group or an alkoxycarbonyl group, or asymmetric hydrogenation of the exo-methylene group of the compound of formula (XI) in the presence of a specific catalyst.

The compounds obtained from the intermediates (VIII) as produced by the above-mentioned processes are, in most cases, mixtures of stereoisomers, i.e., an α-isomer and a β-isomer, in a specific ratio.

As stated above, a number of special phosphine compounds have so far been prepared in order to get an active catalyst for asymmetric hydroformylation. However, the known phosphine complex catalysts are not always satisfactory in terms of regio and enantio selectivities. It has therefore been demanded to develop a novel phosphine compound and a transition metal-phosphine complex performing higher regio and enantio selectivities as compared with conventional catalysts.

There has also been a demand to develop an efficient process for producing a compound (VIII) having a

methyl group in β-configuration, which is a versatide as an intermediate for carbapenem antibiotics, or a precursor thereof.

To meet these demands, the present inventors have conducted extensive investigations on various phosphine compounds. It has now been found as a result that a transition metal complex containing a phosphine compound having an optically active biphenol or binaphthol skeleton as a ligand is markedly superior to known transition metal optically active phosphine complexes in regio and enantio selectivities in asymmetric hydroformylation and that use of the complex makes it possible to synthesize a variety of optically active aldehydes with ease and particularly a compound which can easily lead to the above compound (VIII) which is an important intermediate for carbapenem antibiotics.

The following documents: Tetrahedron letters, 1988, 29, 6461-6464; Chemistry letters, 1989, 445-448; Tetrahedron letters, 1988, 44, 2149-2165; Tetrahedron letters, 1986, 27, 6241-6244; JP 62174048; Chem. Pharm. Bull, 1992, 40, 1098-1104; EP 188816; and EP 180189 are all concerned with the oxidation of the alcohol compound to the carboxylic acid compound which will proceed via the aldehyde as an intermediate.

EP 113101, EP 89139 and EP 10317 disclose the racemic form of the aldehyde of the present application as an intermediate used in the preparation of carbapenem antibiotics.

The present invention relates to a 4-[(R)-1'-formylethyl]azetidin-2-one derivative represented by formula (VII):

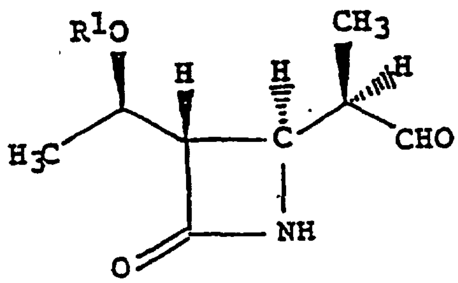

(VII)

wherein $R^1$ represents a hydrogen atom or a hydroxyl-protecting group.

The present invention further relates to a process for preparing a 4- [(R)-1' -formylethyl]azetidin-2-one derivative of formula (VII) comprising hydroformylation of 4-vinylazetidin-2-one derivatives in the presence of a phosphine complex composed of a transition metal and a compound represented by formula (I).

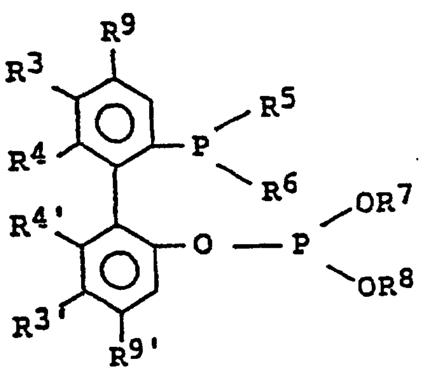

(I)

wherein $R^4$ and $R^{4'}$, which may be the same or different, each represent a hydrogen atom, a lower alkyl group or a lower alkoxy group; $R^3$, $R^{3'}$, $R^9$, and $R^{9'}$, which may be the same or different, each represent a hydrogen atom, a lower alkyl group, a lower alkoxy group or a halogen atom; or a pair of $R^3$ and $R^4$ or a pair of $R^{3'}$ and $R^{4'}$ may form a ring; $R^5$ and $R^6$, which may be the same or different, each represent a phenyl group or a phenyl group substituted with a lower alkyl group, a halogen atom or a lower alkoxy group; and $R^7$ and $R^8$, which may be the same or different, each represent a phenyl group or a phenyl group substituted with a lower alkyl group, a lower alkoxy group or a halogen atom; or $R^7$ and $R^8$ may be taken together to form a divalent hydrocarbon group.

In the present invention, the term "lower alkyl group" includes methyl, ethyl, propyl, isopropyl, butyl, isobutyl, sec-butyl, and t-butyl groups. The term "halogen atom" includes chlorine, bromine, iodine, and fluorine atoms. The

term "lower alkoxy group" includes methoxy, ethoxy, propoxy, isopropoxy, butoxy, isobutoxy, sec-butoxy, and t-butoxy groups. The term "divalent hydrocarbon group" includes biarylene groups, e.g., a substituted or unsubstituted biphenyl group, a substituted or unsubstituted binaphthyl group, a substituted or unsubstituted bianthryl group, and a substituted or unsubstituted biphenanthryl group; and straight chain or branched and saturated or unsaturated alkylene groups having 2-6 carbon atoms, e.g., an ethylene group, a trimethylene group, a tetramethylene group, a pentamethylene group, a 1,4-dimethyltetramethylene group, a 1,3-butadienylene group, and a 1,4-dimethyl-1,3-butadienylene group.

Specific examples of the phosphine compounds according to the present invention are shown in Tables 1 and 2 below. Abbreviations used in the Tables have the following meaning.

binap: 1,1'-binaphthalene-2,2'-diyl group
biphenyl: 1,1'-biphenyl-2,2'-diyl group
OcH-binap: 5,5',6,6',7,7',8,8'-octahydro-1,1'-binaphthalene-2,2'-diyl group
Ph: phenyl group
p-MeO: p-methoxyphenyl group
xylyl: 3,5-xylyl group
MeO: methoxy group
p-t-Bu: p-t-butylphenyl group
p-Cℓ: p-chlorophenyl group
p-Br: p-bromophenyl group
phenanthryl: 10,10'-biphenanthrene-9,9'-diyl

TABLE 1

| No. | $R^5$ | $R^6$ | $R^7$, $R^8$ | $R^4$ or $R^{4'}$ | $R^3$ or $R^{3'}$ | $R^9$ or $R^{9'}$ |
|---|---|---|---|---|---|---|
| 1 | Ph | Ph | binap | H | H | H |
| 2 | Ph | Ph | binap | Me | H | Me |
| 3 | Ph | Ph | binap | MeO | H | MeO |
| 4 | Ph | Ph | binap | Me | Cℓ | Me |
| 5 | Ph | Ph | OcH-binap | H | H | H |
| 6 | Ph | Ph | OcH-binap | Me | H | Me |
| 7 | Ph | Ph | biphenyl | Me | Cℓ | Me |
| 8 | Ph | Ph | biphenyl | MeO | H | MeO |
| 9 | Ph | Ph | Ph, Ph | H | H | H |
| 10 | Ph | Ph | p-Cℓ, p-Cℓ | Me | H | Me |
| 11 | p-tol | p-tol | binap | H | H | H |
| 12 | p-tol | p-tol | binap | MeO | H | MeO |
| 13 | p-tol | p-tol | OcH-binap | MeO | Cℓ | MeO |
| 14 | p-tol | p-tol | biphenyl | H | H | H |
| 15 | p-MeO | p-MeO | binap | MeO | H | MeO |
| 16 | p-MeO | p-MeO | OcH-binap | Me | Cℓ | Me |
| 17 | xylyl | xylyl | binap | H | H | H |
| 18 | p-t-Bu | p-t-Bu | binap | H | H | H |
| 19 | p-Cℓ | p-Cℓ | binap | Me | H | Me |
| 20 | p-Cℓ | p-Cℓ | p-t-Bu, p-t-Bu | MeO | Cℓ | MeO |
| 21 | p-Br | p-Br | binap | Me | H | Me |

TABLE 2

| No. | 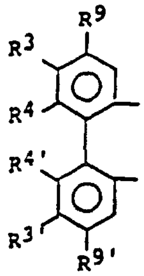 | $R^7$, $R^8$ | $R^5$ | $R^6$ |
|---|---|---|---|---|
| 22 | binap | Ph, Ph | Ph | Ph |
| 23 | binap | Ph, Ph | xylyl | xylyl |
| 24 | binap | binap | Ph | Ph |
| 25 | binap | binap | p-Cℓ | p-Cℓ |
| 26 | binap | binap | p-t-Bu | p-t-Bu |
| 27 | binap | binap | xylyl | xylyl |
| 28 | binap | binap | p-MeO | p-MeO |
| 29 | binap | biphenyl | Ph | Ph |
| 30 | binap | biphenyl | p-Cℓ | p-Cℓ |
| 31 | binap | biphenyl | xylyl | xylyl |
| 32 | binap | OcH-binap | Ph | Ph |
| 33 | binap | OcH-binap | p-MeO | p-MeO |
| 34 | binap | OcH-binap | xylyl | xylyl |
| 35 | binap | phenanthryl | Ph | Ph |
| 36 | OcH-binap | binap | Ph | Ph |
| 37 | OcH-binap | binap | p-Cℓ | p-Cℓ |
| 38 | OcH-binap | binap | p-t-Bu | p-t-Bu |
| 39 | OcH-binap | binap | xylyl | xylyl |
| 40 | OcH-binap | biphenyl | Ph | Ph |
| 41 | OcH-binap | biphenyl | p-Cℓ | p-Cℓ |
| 42 | OcH-binap | biphenyl | xylyl | xylyl |
| 43 | OcH-binap | OcH-binap | Ph | Ph |
| 44 | OcH-binap | OcH-binap | p-MeO | p-MeO |
| 45 | OcH-binap | OcH-binap | xylyl | xylyl |
| 46 | OcH-binap | phenanthryl | Ph | Ph |
| 47 | OcH-binap | phenanthryl | p-Cℓ | p-Cℓ |

The phosphine compound (I) of the present invention includes optically active compounds and non-optically active compounds all of which fall within the scope of the present invention.

The phosphine compound (I) can be synthesized by, for example, the following reaction route:

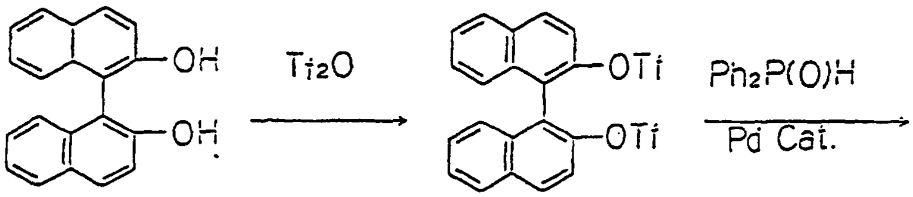


1 2

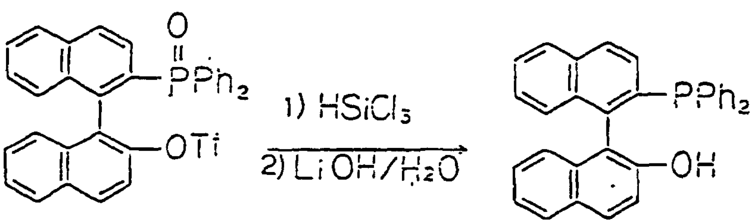


3 4

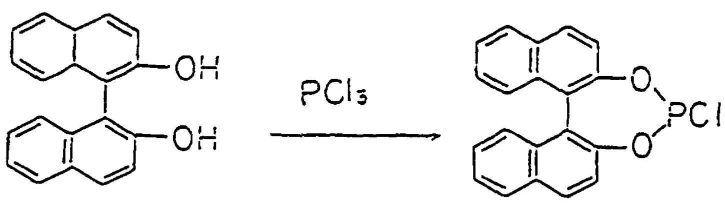


1 5

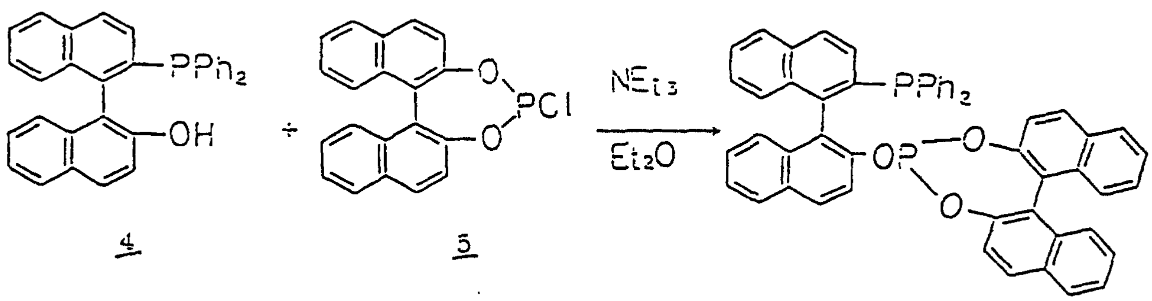


4 5

6

wherein Tf represents a trifluoromethanesulfonyl group; Ph represents a phenyl group; and Et represents an ethyl group.

More specifically, 1,1'-bi-2-naphthol 1 is reacted with trifluoromethanesulfonic acid anhydride to obtain 2,2'-bis(trifluoromethanesulfonyloxy)-1,1'-binaphthyl 2 which is then reacted with diphenylphosphine oxide in the presence

of a palladium complex catalyst to obtain 2-diphenylphosphinyl-2'-trifluoromethanesulfonyloxy-1,1'-binaphthyl 3. The compound 3 is reduced by trichlorosilane in the presence of triethylamine and then hydrolyzed to obtain 2-diphenylphosphino-2'-hydroxy-1,1'-binaphthyl 4. The compound 4 is reacted with a separately synthesized chlorophosphite 5 in the presence of triethylamine to obtain phosphine compound 6 of the present invention.

The phosphine compound (I) according to the present invention serves as a ligand in the formation of complexes with transition metals. The transition metals capable of forming a complex with the compound (I) include rhodium, ruthenium, iridium, and platinum. Of these, rhodium is preferred. Of the transition metal complexes according to the present invention, the rhodium complex, for example, can be synthesized by reacting the phosphine compound (I) with a rhodium compound in an appropriate solvent. The specific examples of the solvent include methylene chloride, hydrocarbons (e.g., toluene, benzene, hexane, heptane, isooctane, decane, etc.), ethers (e.g., diethylether, tetrahydrofuran, etc.), ketones (e.g., acetone, methyl ethyl ketone, etc.), lower alcohols (methanol, ethanol, etc), and esters (e.g., ethyl acetate). Of these, methylene chloride and toluene are preferred. The rhodium compound as a complex precursor includes $RhC\ell_3$, $RhBr_3$, $RhI_3$, $Rh_2O_3$, $Rh_2(OAc)_3$ (Ac represents an acetyl group, hereinafter the same), $[Rh(O_2C_7H_{15})_2]_2$, $Rh(acac)_3$ (acac represents acetylacetonato, hereinafter the same), $Rh_4(CO)_{12}$, $Rh_6(CO)_{16}$, $[Rh(COD)C\ell]_2$ (COD represents 1,5-cyclooctadiene, hereinafter the same), $[Rh(COD)Br]_2$, $[Rh(COD)I]_2$, $[Rh(COD)OAc]_2$, $[Rh(COD)OCOC(CH_3)_3]_2$, $[Rh(NBD)C\ell]_2$ (NBD represents norbornadiene, hereinafter the same), $[Rh(NBD)Br]_2$, $[Rh(NBD)I]_2$, $[Rh(NBD)OAc]_2$, $[Rh(NBD)OCOC(CH_3)_3]_2$, Rh(COD)(acac), Rh(NBD)(acac), $Rh(CO)_2(acac)$, $[Rh(CO)_2C\ell]_2$, $[Rh(CO)_2Br]_2$, $[Rh(CO)_2I]_2$, $Rh(CO)_2(Cp)$ (Cp represents 1,3-cyclopentadiene), $Rh(CO)_2(tmCp)$ (tmCp represents 1,2,3,4-tetramethyl-1,3-cyclopentadiene, hereinafter the same), $[Rh(CO)(tmCp)_2]$, $Rh(C_2H_4)_2(acac)$, $[Rh(C_2H_4)_2C\ell]_2$, $[Rh(C_2H_4)_2Br]_2$, $[Rh(C_2H_4)_2I]_2$, $[Rh(C_2H_4)_2(tmCp)]$, $RhC\ell(PPh_3)_3$ (Ph represents a phenyl group, hereinafter the same), $RhBr(PPh_3)_3$, $RhI(PPh_3)_3$, $RhH(CO)(PPh_3)_3$, $RhH(PPh_3)_3$, $RhH(P(i\text{-}Pr)_3)_3$ (i-Pr represents an isopropyl group, hereinafter the same), $RhHC\ell_2(PPh_3)_2$, $RhHC\ell_2(AsPh_3)_2$, $RhHC\ell_2(SbPh_3)_2$, $RhH_2C\ell(PPh_3)_2$, $RhH_2Br(PPh_3)_2$, $RhH_2I(PPh_3)_2$, $Rh(OAc)(CO)(PCp_3)_2$, $Rh(OCOPh)(CO)(PCp_3)_2$, $Rh(C\ell O_4)(CO)(PCp_3)_2$, $Rh(C\ell)(CO)(PCp_3)_2$, $Rh(C\ell)(CO)(PBu_3)_2$, $Rh(C\ell)(CO)(PPh_3)_2$, $Rh(I)(CO)(PCp_3)_2$, $[RhH\{P(O\text{-}i\text{-}Pr)_3\}_2]_2$, $Rh(C_5H_7O_2)(C_2H_4)$, $[Rh(COD)_2]BF_4$, $[Rh(COD)(CH_3CN)_2]BF_4$, $[Rh(COD)(Ph_2P(CH_2)_4PPh_3)]BF_4$, $[Rh(PhCH_3)(Ph_2P(CH_2)_5PPh_2)]BF_4$, $[Rh(PhCH_3)\{PPh_2(o\text{-}CH_3OC_6H_4)_2\}_2]BF_4$, $[Rh(COD)(PPh_3)_2]PF_6$, $[Rh(COD)(AsPh_3)_2]C\ell O_4$, $[Rh(NBD)(PPh_3)_2]C\ell O_4$, $[RhH_2(PPh_3)_2(AcCH_3)(C_2H_5OH)]C\ell O_4$, $[RhH_2(PPh_3)_2(CH_3CN)_2]C\ell O_4$, $[RhH_2(PPh_3)_2(C_2H_5OH)_2]C\ell O_4$, $[RhC\ell_2(\eta\text{-}C_5H_{10}(CH_3)_4)]_2$, $Rh(Cp)(PPh_3)_2$, $[Rh(tmCp)(CH_3CN)_3](PF_6)_2$, $Rh_2C\ell_2(\eta\text{-}C_3H_5)_4$, and $Rh(\eta\text{-}C_3H_5)_3$.

The rhodium-phosphine complexes usually have one rhodium atom in the molecule thereof (mononuclear complexes) and in some cases have two or more rhodium atoms (polynuclear complexes).

Ruthenium complexes, iridium complexes and platinum complexes containing the phosphine compound (I) as a ligand can also be prepared in the same manner as described above.

The transition metal-phosphine complexes of the present invention are useful as a catalyst for asymmetric hydroformylation to produce optically active aldehydes.

Optically active aldehydes can also be prepared by hydroformylation of olefins in the presence of the phosphine compound (I). That is, hydroformylation of an olefin proceeds in the presence of a transition metal compound and the phosphine compound (I) separately added and mixed in a reaction solvent to form a transition metal-phosphine complex in situ thereby to produce an optically active aldehyde similarly to the case of using a previously prepared transition metal-phosphine complex. This will be proved from the following fact.

Where a transition metal compound, e.g., $Rh(CO)_2(acac)$ and the phosphine compound (hereinafter abbreviated as "phos") are reacted at an equivalent ratio in a solvent, e.g., methylene chloride, ligand exchange takes place between two carbonyl groups and phos to form an Rh(acac)(phos) type complex. On $^{31}P$-NMR analysis of the isolated complex, there are revealed two signals. Where one equivalent of $Rh(CO)_2(acac)$ and 2.5 equivalents of phos are similarly reacted in a solvent, the $^{31}P$-NMR spectrum shows two signals assigned to phos per se in addition to those to the complex. On the other hand, the system containing one equivalent of $Rh(CO)_2(acac)$ and 2.5 equivalents of phos under hydroformylation reaction conditions (before addition of carbon monoxide and hydrogen) similarly shows signals both of the complex and phos. From these results, it is seen that the reaction necessarily proceeds via formation of Rh(acac)(phos).

Illustrative examples of olefins which can be used as a reaction substrate in the present invention include vinyl chloride, vinyl bromide, vinyl iodide, 1-propene, 1-butene, 1-pentene, 1-hexene, 3,3-dimethyl-1-butene, N-vinylphthalimide, vinyl acetate, vinyl propionate, vinyl valerate, acrylonitrile, acrylic acid, methyl acrylate, ethyl acrylate, butyl acrylate, t-butyl acrylate, methyl vinyl ether, ethyl vinyl ether, butyl vinyl ether, t-butyl vinyl ether, 3-buten-2-one, 1-buten-3-one, 1-hepten-3-one, 4,4-dimethyl-1-penten-3-one, 1-phenyl-2-propen-l-one, N-vinylacetamide, N-vinylbenzamide, methylvinylamine, ethylvinylamine, butylvinylamine, dimethylvinylamine, diethylvinylamine, dibutylvinylamine, styrene, chlorostyrene, bromostyrene, methylstyrene, 4-t-butylstyrene, 4-isobutylstyrene, methoxystyrene, ethoxystyrene, vinylnaphthalene, 2-methoxy-6-vinylnaphthalene, 1-chloro-1-propene, 1-bromo-1-propene, 2-butene, 2-pentene, 2-hexene, 2-heptene, 1-propenyl acetate, 1-propenyl cyanide, crotonic acid, methyl crotonate, butyl crotonate, methyl 1-propenyl ether, 3-penten-2-one, 2-octen-4-one, N-2-pentenylacetamide, methyl 2-propenylamine, butyl

2-propenylamine, dimethyl 2-propenylamine, dibutyl 2-propenylamine, 4-(2-propenyl)chlorobenzene, 4-(2-propenyl) toluene, 4-(2-propenyl)butylbenzene, (1'R,3S,4R)-3-(1'-(Pro-1-)oxy)ethyl-4-vinylazetizin-2-one, indene, and 1,2-dihydronaphthalene.

"Pro-1" as used above corresponds to $R_1$ in formula (IV), including a hydrogen atom and a hydroxyl-protecting group. Examples of the hydroxyl-protecting group include substituted silyl groups, e.g., trimethylsilyl, triethylsilyl, isopropyldimethylsilyl, t-butyldimethylsilyl, (triphenylmethyl)dimethylsilyl, t-butyldiphenylsilyl, methyldiisopropylsilyl, methyl-di-t-butylsilyl, tribenzylsilyl, tri(p-tolyl)silyl, triisopropylsilyl, and triphenylsilyl; acyl groups, e.g., formyl, benzoylformyl, acetyl, chloroacetyl, dichloroacetyl, trichloroacetyl, trifluoroacetyl, methoxyacetyl, phenoxyacetyl, p-chlorophenoxyacetyl, benzoyl, benzyloxycarbonyl, and ethoxycarbonyl; and aralkyl groups, e.g., benzyl, benzhydryl, and triphenylmethyl. $R_1$ is preferably a hydrogen atom, a trimethylsily group, a t-butyldimethylsilyl group, a benzyloxycarbonyl group, an ethoxycarbonyl group, an acetyl group, a benzyl group, a benzhydryl group, and a triphenylmethyl group.

The hydroformylation can be carried out by adding to a reaction system the phosphine compound (I) and a compound of a transition metal selected from rhodium, ruthenium, iridium, and platinum separately or the transition metal-phosphine complex previously formed from a compound of a transition metal selected from rhodium, ruthenium, iridium, and platinum and the phosphine compound (I). The complex catalyst or catalyst system is added in an amount of preferably from 0.0001 to 1000 mg, and more preferably from 0.001 to 100 mg, per liter of the liquid phase in terms of the transition metal atom. The phosphine compound (I) is preferably used in an amount 1 to 5 times, and more preferably 2 to 4 times, the mole number of the metal atom.

While the hydroformylation may be effected in the absence of a reaction solvent, use of a reaction solvent is generally recommended. The reaction solvent to be used is not particularly limited as long as it does not adversely affect the reaction. Hydrocarbon solvents, e.g., hexane, heptane, octane, isooctane, nonane, decane, cyclohexane, cyclopentane, benzene, toluene, xylene, and mesitylene, are especially preferred. In addition, ethers, e.g., diisopropyl ether, dibutyl ether, tetrahydrofuran, dimethoxyethane, and diethylene glycol dimethyl ether; ketones, e.g., diisobutyl ketone, methyl isobutyl ketone, acetone, and methyl ethyl ketone; esters, e.g., ethyl acetate, butyl butyrate, and butyl benzoate; and alcohols, e.g., methanol, ethanol, butanol, and t-butanol; are also usable. These solvents may be used either individually or in combination thereof.

It is generally preferred to conduct hydroformylation in the presence of water in order to enhance the catalytic activity. In the present invention, too, water may be present in the reaction system. While the amount of water to be added is not limited, an extremely small amount of water produces no essential effect, and an extremely large amount of water adds no further improvement. An increase of reaction rate is often obtained by addition of water in an amount 0.001 to 1000 times the weight of the olefin substrate.

For the purpose of improving catalytic activity, regio and enantio selectivities, various additives other than water may be added to the reaction system. For example, presence of phosphorus compounds, e.g., triethylphosphine oxide, triphenylphosphine oxide, tributylphosphine oxide, triethyl phosphite, tributyl phosphite, and triphenyl phosphite; or carboxylic acids, e.g., acetic acid, propionic acid and pivalic acid, in the reaction system gives rise to no interference with the reaction.

The hydroformylation temperature usually ranges from -20° to 250°C, and preferably from 10° to 150°C. Within this range, low temperatures are preferred from the standpoint of thermal stability of the resulting aldehyde, while high temperatures are preferred from the viewpoint of reaction rate. The reaction pressure usually ranges from 5 to 200 kg/cm$^2$, and preferably from 20 to 150 kg/cm$^2$. Carbon monoxide and hydrogen are usually fed to the reaction system at a molar ratio of from 0.1 to 10, and preferably from 0.2 to 4. As far as the carbon monoxide to hydrogen molar ratio falls within this range, the mixed gas may be diluted with an inert gas, such as methane, nitrogen, argon, helium, and carbon dioxide, either individually or in combination thereof.

According to the present invention, a 4-[(R)-1'-formylethyl]azetidin-2-one derivative represented by formula (VII):

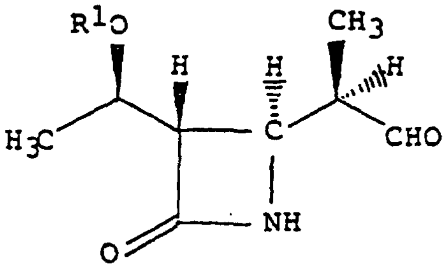

(VII)

wherein $R^1$ is as defined above,
can be prepared in excellent optical purity with high regioselectivity by subjecting a compound represented by formula (VI):

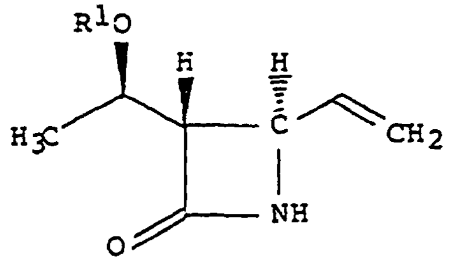

(VI)

wherein $R^1$ is as defined above,
to the above-mentioned hydroformylation process.

The starting compound of formula (VI) is obtained, for example, by the process described in <u>Liebig Ann. Chem.,</u> pp. 539-560 (1974) as follows. A 4-acetoxyazetidin-2-one derivative represented by formula (XII):

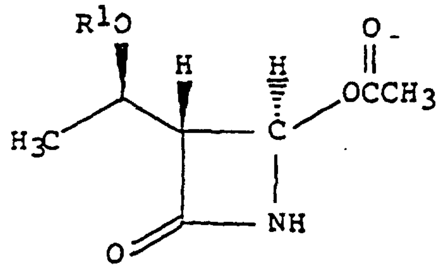

(XII)

wherein $R^1$ is as defined above,
is reacted with a sodium, potassium or lithium salt of benzenesulfinic acid or p-toluenesulfinic acid in a soluble solvent, e.g., acetone-water, methanol or water-methanol, to obtain a compound represented by formula (XIII):

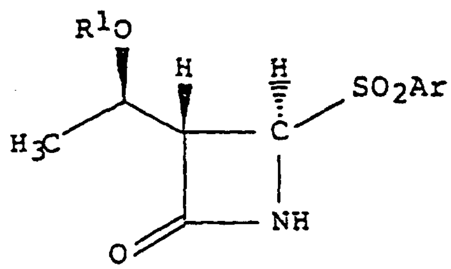

(XIII)

wherein $R^1$ is as defined above; and Ar represents a phenyl group which may be substituted with a halogen atom, a lower alkyl group, etc.
The compound (XIII) is then reacted with an organic vinyl compound as a vinylating agent, such as vinylmagnesium chloride, vinylmagnesium bromide, vinylmagnesium iodide, divinylmagnesium, vinyllithium, vinylzinc chloride or divinylzinc, in accordance with the procedure described in <u>J. Chem. Soc. Chem. Commn.</u>, pp. 736-737 (1980) to obtain the compound (VI).

The compound (VII) can be obtained by hydroformylation of the thus prepared compound (VI) in the presence of the transition metal-phosphine complex of the present invention or both the phosphine compound (I) and a transition metal compound.

It is essentially required for obtaining the compound (VII) in which the 1'-formyl group has an R-configuration, i.e., a β-compound that the skeleton moiety of the phosphine compound (I) should have an R-configuration and that the phosphite moiety as a counterpart, when derived from an axially dissymmetric diol, should have an S-configuration.

The formyl group of the compound (VII) can easily be converted to a carboxyl group by an ordinary oxidation reaction, for example, Jones oxidation to provide the compound of formula (VIII), an important intermediate for carbapenem antibiotics.

According to the present invention, optically active aldehydes useful as pharmaceuticals, agricultural chemicals, perfumes, etc. or intermediates therefor can be synthesized in a high optical purity.

The present invention is now illustrated in greater detail with reference to Examples, but it should be understood that the present invention is not construed as being limited thereto. All the parts and percents are by weight unless otherwise specified.

Measuring instruments used in Examples are as follows.

Nuclear Magnetic Resonance Spectrum (NMR):

Model EX-270 manufactured by JEOL Ltd.
Model AM-400 manufactured by Bruker, Inc.
Internal Standard:
$^{1}$H-NMR: tetramethylsilane
External Standard:
$^{31}$P-NMR: 85% phosphoric acid

Optical Purity:

GC-15A Gas Chromatograph manufactured by Shimadzu Corporation
Column: Chiral capillary column, CHROMPACK β-236M

Chemical Purity:

Hitachi 263-30 Gas Chromatograph manufactured by Hitachi, Ltd.

Optical Rotation:

Model DIP-360 manufactured by JASCO Inc.

EXAMPLE 1

Preparation of (S)-3,3'-Dichloro-2,2',4,4'-tetramethyl-6-diphenylphosphinobiphenyl-6'-yloxy((R)-1,1'-binaphthalene-2,2'-diyldioxy)phosphine and (R)-3,3'-Dichloro-2,2',4,4'-tetramethyl-6-diphenylphosphinobiphenyl-6'-yloxy((R)-1,1'-binaphthalene-2,2'-diyldioxy)phosphine

(1) Preparation of (±)-3,3'-Dichloro-2,2',4,4'-tetramethylbiphenyl-6,6'-diol (hereinafter referred to as compound (A)):

In a flask were charged 10.1 g (70.0 mmol) of 4-chloro-3,5-xylenol and 37.8 g (140 mmol) of iron trichloride hexahydrate, and the mixture was allowed to react at 80°C for 16 hours. After completion of the reaction, the reaction mixture was treated with 300 mℓ of 1N hydrochloric acid and extracted three 250 mℓ portions of dichloromethane. The organic layer was washed successively with 500 mℓ of a saturated sodium hydrogencarbonate aqueous solution and 500 mℓ of brine, concentrated, and recrystallized from hexane/chloroform to obtain 3.32 g (percent yield 30%) of compound (A).

Melting point: 231-233°C

$^{1}$H-NMR ($CDC\ell_3$) δ: 2.05 (s, 6H), 2.41 (s, 6H), 4.57 (s, 2H), 6.84 (s, 2H)

(2) Preparation of (±)-3,3'-Dichloro-2,2',4,4'-tetramethyl-6,6'-bis(trifluoromethanesulfonyloxy)biphenyl (hereinafter referred to as compound (B)):

In a flask, 6.22 g (20.0 mol) of compound (A) obtained in (1) above, 4.72 g (44.0 mmol) of 2,6-lutidine, and 732 mg (6.00 mmol) of 4-dimethylaminopyridine were dissolved in 30 mℓ of dichloromethane in an argon stream, and 12.4 g (44.0 mmol) of trifluoromethanesulfonic acid anhydride was added to the solution at 0°C, followed by allowing to react at that temperature for 1 hour. After completion of the reaction, the solvent was removed under reduced pressure, and the residue was purified by column chromatography (hexane/chloroform=1:1 by volume) to obtain 10.9 g (percent yield 95%) of compound (B).
Melting point: 90-91°C
$^1$H-NMR ($CDC\ell_3$) δ: 2.14 (s, 6H), 2.50 (s, 6H), 7.17 (s, 2H)

(3) Preparation of (±)-3,3'-Dichloro-2,2',4,4'-tetramethyl-6-diphenylphosphinyl-6'-trifluoromethanesulfonyloxybiphenyl (hereinafter referred to as compound (C)):

In a flask, 11.44 g (19.9 mmol) of compound (B) obtained in (2) above and 8.05 g (39.8 mmol) of diphenylphosphine oxide and 223 mg (0.99 mmol) of palladium acetate were dissolved in 980 mℓ of dimethyl sulfoxide in an argon stream. To the solution were added 821 mg (1.99 mmol) of 1,3-bis(diphenylphosphino)propane and 15.3 mℓ of diisopropylamine over 20 minutes, followed by stirring at 90°C for 60 hours. After completion of the reaction, 120 mℓ of water was added to the reaction mixture, and the mixture was extracted with two 250 mℓ portions of ethyl ether. The organic layer was washed successively with 200 mℓ of water, 200 mℓ of 1N hydrochloric acid, 200 mℓ of a saturated sodium hydrogencarbonate aqueous solution, and 200 mℓ of brine and dried over magnesium sulfate. Purification by column chromatography (hexane/ethyl acetate=1:1 by volume) gave 7.77 g (percent yield 62%) of compound (C).
Melting point: 159-160°C
$^{31}$P-NMR ($CDC\ell_3$) δ: 27.75

(4) Preparation of (±)-3,3'-Dichloro-2,2',4,4'-tetramethyl-6-diphenylphosphino-6'-hydroxybiphenyl (hereinafter referred to as compound (D)):

In a flask, 7.32 g (11.7 mmol) of compound (C) obtained in (3) above and 47.2 g (467 mmol) of triethylamine were dissolved in 400 mℓ of xylene. To the solution was added dropwise 63.3 g (467 mmol) of trichlorosilane at 0°C, followed by stirring at 120°C for 35 hours. To the solution was carefully added dropwise 200 mℓ of a saturated sodium hydroxide aqueous solution at 0°C, followed by stirring at 60°C for 2 hours. After completion of the reaction, the reaction mixture was extracted with two 150 mℓ portions of toluene. The organic layer was washed with 400 mℓ of water and then with 300 mℓ of brine and dried over magnesium sulfate. The solvent was removed under reduced pressure. The residue weighing 7.46 g was dissolved in 130 mℓ of tetrahydrofuran in a flask, and 44 mℓ of an aqueous solution of 5.87 g (140 mmol) of lithium hydroxide monohydrate was added thereto, followed by stirring at 30°C for 15 hours. After completion of the reaction, 200 mℓ of 1N hydrochloric acid was added to the reaction mixture, and the mixture was extracted with two 200 mℓ portions of ethyl ether. The resulting organic layer was washed successively with 300 mℓ of water and 250 mℓ of brine and dried over magnesium sulfate. The solvent was removed under reduced pressure, and the residue was purified by column chromatography (hexane/ethyl acetate=12:1 to 3:1 by volume) to obtain 5.05 g of compound (D) in 90% yield based on compound (C).
Melting point: 71-79°C
$^{31}$P-NMR ($CDC\ell_3$) δ: -13.37

(5) Preparation of (R)-1,1'-Binaphthalene-2,2'-diyldioxychlorophosphine (hereinafter referred to as compound (E)) and (S)-1,1'-binaphthalene-2,2'-diyldioxychlorophosphine (hereinafter referred to as compound (F)):

In 50 mℓ-flask equipped with a reflux condenser were charged 4.94 g (17.3 mmol) of (R)-1,1'-bi-2-naphthol and 104 g (757 mmol) of phosphorus trichloride in an argon stream, and the mixture was refluxed for 17 hours. After cooling to room temperature, the unreacted phosphorus trichloride was removed by distillation under reduced pressure. The residue was subjected twice to azeotropic distillation together with 50 mℓ of toluene under reduced pressure. The residue was dissolved in benzene, and the solution was dried under reduced pressure to obtain 5.56 g (percent yield 92%) of compound (E) as a white solid.
$^{31}$P-NMR ($CDC\ell_3$) δ: 178.8
Compound (F) was prepared quantitatively in the same manner as for compound (E).

(6) Preparation of (S)-3,3'-Dichloro-2,2',4,4'-tetramethyl-6-diphenylphosphinobiphenyl-6'-yloxy((R)-1,1'-binaphthalene-2,2'-diyldioxy)phosphine (hereinafter referred to as (S,R)-biphemphos) and (R)-3,3'-dichloro-2,2',4,4'-tetramethyl-6-diphenylphosphinobiphenyl-6'-yloxy((R)-1,1'-binaphthalene-2,2'-diyldioxy)phosphine (hereinafter referred to as (R,R)-biphemphos):

In 40 mℓ of toluene were dissolved 756 mg (1.58 mmol) of compound (D) and 1336 mg (3.81 mmol) of compound (E) in a flask, and 386 mg (3.81 mmol) of triethylamine was added thereto at 0°C. After stirring the mixture at room temperature for 42 hours, 50 mℓ of water was added thereto to cease the reaction. To the reaction mixture was

added 50 mℓ of ethyl ether for liquid-liquid separation, and the organic layer was washed successively with two 80 mℓ portions of water and 80 mℓ of brine, and dried over anhydrous magnesium sulfate. The solvent was removed under reduced pressure, and the resulting crude product was purified by column chromatography (hexane/dichloromethane=20:1 to 3:1 by volume) to obtain 395 mg (percent yield 32%) of (S,R)-biphemphos and 259 mg (percent yield 21%) of (R,R)-biphemphos.

(S,R)-biphemphos:

Melting point: 155-162°C
$[\alpha]_D^{25}$: -281° (c=1.0, toluene)
$^{31}P$-NMR (toluene-$d_8$) δ: -13.4 (d, $J_{p-p}$=35.14Hz), 146.7 (d) Thin layer chromatography (TLC):
Rf=0.47 (hexane/dichloromethane=1:1 by volume)

(R,R)-biphemphos:

Melting point: 153-159°C
$[\alpha]_D^{22}$: -252° (c=1.0, toluene)
$^{31}P$-NMR (toluene-$d_8$) δ: -12.6 (d, $J_{p-p}$=12.2Hz), 145.8 (d) TLC: Rf=0.42 (hexane/dichloromethane=1:1 by volume)

EXAMPLE 2

(R)-3,3'-Dichloro-2,2',4,4'-tetramethyl-6-diphenylphosphinobiphenyl-6'-yloxy((S)-1,1'-binaphthalene-2,2'-diyldioxy)phosphine (hereinafter referred to as (R,S)-biphemphos) and (S)-3,3'-dichloro-2,2',4,4'-tetramethyl-6-diphenylphosphinobiphenyl-6'-yloxy((S)-1,1'-binaphthalene-2,2'-diyldioxy)phosphine (hereinafter referred to as (S,S)-biphemphos), which are isomers of the products of Example 1, were obtained in the same manner as in Example 1.

(R,S)-biphemphos:

Melting point: 153-158°C
$[\alpha]_D^{21}$: 273° (c=1.0, toluene)
$^{31}P$-NMR (toluene-$d_8$) δ: -13.3 (d, $J_{P-P}$=36.6Hz), 146.8 (d) TLC: Rf=0.47 (hexane/dichloromethane=1:1 by volume)

(S,S)-biphemphos:

Melting point: 147-151°C
$[\alpha]_D^{21}$: 253° (c=1.0, toluene)
$^{31}P$-NMR (toluene-$d_8$) δ: -12.6 (d, $J_{P-P}$=13.8Hz), 145.8 (d)
TLC: Rf=0.42 (hexane/dichloromethane=1:1 by volume)

EXAMPLE 3

Preparation of (R)-3,3',4,4',5,5'-hexamethyl-6-diphenylphosphinobiphenyl-6'-yloxy((S)-1,1'-binaphthalene-2,2'-diyldioxy)phosphine and (S)-3,3',4,4',5,5'-hexamethyl-6-diphenylphosphinobiphenyl -6'-yloxy((S)-1,1'-binaphthalene-2,2'-diyldioxy)phosphine

(1) Preparation of (±)-2,2',3,3',4,4'-hexamethylbiphenyl-6,6'-diol (hereinafter referred to as compound (A"):

Compound (A") was prepared from 3,4,5-trimethylphenol in 53% yield by the same method of the synthesis of compound (A).
$^1H$-NMR ($CDC\ell_3$) δ: 1.92 (s,6H), 2.16 (s,6H), 2.31 (s,6H), 4.50 (s,2H), 6.74 (s,2H)
TLC: Rf=0.28 (dichloromethane)

(2) Preparation of (±)-2,2',3,3',4,4'-hexamethyl-6,6'-bis(trifluoromethanesulfonyloxy)biphenyl (hereinafter referred to as compound (B")):

Compound (B") was prepared from compound (A") in 90% yield by the same method of the synthesis of compound (B).
$^1H$-NMR ($CDC\ell_3$) δ: 2.00 (s,6H), 2.24 (s,6H), 2.38 (s,6H), 7.04 (s,2H)

TLC: Rf=0.31 (hexane/dichloromethane=1:1 by volume)

(3) Preparation of (±)-2,2',3,3',4,4'-hexamethyl-6-diphenylphosphinyl-6'-trifluoromethanesulfonyloxybiphenyl (hereinafter referred to as compound (C")):

Compound (C") was also prepared from compound (B") in 77% yield by the same method of the synthesis of compound (C).
$^{31}$P-NMR ($CDC\ell_3$) δ: 28.20
TLC: Rf=0.70 (ethylacetate)

(4) Preparation of (±)-2,2',3,3',4,4'-hexamethyl-6-diphenylphosphino-6'-hydroxybiphenyl (hereinafter referred to as compound (D")):

Compound (D") was prepared from compound (C") in 74% yield by the same method of the synthesis of compound (D).
$^{31}$P-NMR ($CDC\ell_3$) δ: -13.46
TLC: Rf=0.68 (hexane/ethylacetate=7:3)

(5) Preparation of (R)-3,3',4,4',5,5'-hexamethyl-6-diphenylphosphinobiphenyl-6'-yloxy((S)-1,1'-binaphthalene-2,2'-diyldioxy)phosphine (hereinafter referred to as (R,S)-Me-biphemphos) and (S)-3,3',4,4',5,5'-hexamethyl-6-diphenylphosphinobiphenyl-6'-yloxy((S)-1,1'-binaphthalene-2,2'-diyldioxy)phosphine (hereinafter referred to as (S, S)-Me-biphemphos)

(R,S)-Me-biphemphos and (S,S)-Me-biphemphos were prepared by the same method of the synthesis of (R, R)-biphemphos and (S,R)-biphemphos from compound (D") and compound (F).

(R,S)-Me-biphemphos :

$^{31}$P-NMR (benzene-$d_6$) δ: -13.3 (d,Jp-p=39.6Hz), 147.6 (d)
TLC: Rf=0.60 (hexane/dichloromethane=1:1)

(S,R)-Me-biphemphos:

$^{31}$P-NMR (benzene-$d_6$) δ: -12.9 (d,Jp-p=13.8Hz), 147.3 (d)
TLC: Rf=0.53 (hexane/dichloromethane=1:1)

EXAMPLE 4

Preparation of (R)-2-Diphenylphosphino-1,1'-biphenyl-2'-yloxy((R)-1,1'-binaphthalene-2,2'-diyldioxy)-phosphine and (S)-2-Diphenylphosphino-1,1'-biphenyl-2'-yloxy((R)-1,1'-binaphthalene-2,2'-diyldioxy)phosphine

(1) Preparation of 2,2'-bis(trifluoromethanesulfonyloxy)biphenyl (hereinafter referred to as compound (B')):

In the same manner as in Example 1-(2), compound (B') was obtained in 96% yield.
Melting point: 33-34°C
TLC: Rf=0.67 (hexane/ethyl acetate=7:3 by volume)

(2) Preparation of 2-diphenylphosphinyl-2'-trifluoromethanesulfonyloxybiphenyl (hereinafter referred to as compound (C')):

In the same manner as in Example 1-(3), compound (C') was obtained in 79% yield.
Melting point: 123-124°C
$^{31}$P-NMR ($CDC\ell_3$) δ: 27.78
$^{19}$F-NMR ($CDC\ell_3$) δ: -6.05

(3) Preparation of 2-diphenylphosphino-2'-hydroxybiphenyl (hereinafter referred to as compound (D')):

In the same manner as in Example 1-(4), compound (D') was obtained in 60% yield.

Melting point: 122-123°C
$^{31}$P-NMR ($CDC\ell_3$) 6: -12.01

(4) Preparation of (R)-2-diphenylphosphino-1,1'-biphenyl-2'-yloxy((R)-1,1'-binaphthalene-2,2'-diyldioxy)phosphine (hereinafter referred to as (R,R)-H-biphemphos) and (S)-2-diphenylphosphino-1,1'-biphenyl-2'-yloxy((R)-1,1'-binaphthalene-2,2'-diyldioxy)phosphine (hereinafter referred to as (S,R)-H-biphemphos):

In the same manner as in Example 1-(6), 871 mg (percent yield 62%) of a mixture of (R,R)-H-biphemphos and (S,R)-H-biphemphos was obtained at an (S,R)-H-biphemphos/(R,R)-H-biphemphos ratio of 55:45.

Mixture:

Melting point: 150-156°C
$[\alpha]_D^{22}$: 324° (c=1.0, toluene)

(S,R)-H-biphemphos:

$^{31}$P-NMR (toluene-$d_8$) δ: -11.9 (d, $J_{p-p}$=35.1Hz), 146.77 (d)

(R,R)-H-biphemphos:

$^{31}$P-NMR (toluene-$d_8$) δ: -11.5 (d, $J_{p-p}$=21.4Hz), 146.81 (d)

EXAMPLE 5

Synthesis of (R)-2-Diphenylphosphino-5,5',6,6',7,7',8,8'-octahydro-1,1'-binaphthalen-2'-yloxy-((S)-1,1'-binaphthalene-2,2'-diyldioxy)phosphine

(1) Synthesis of (R)-5,5',6,6',7,7',8,8'-octahydro-1,1'-bi-2-naphthol:

In a 200 mℓ autoclave were charged 3.00 g (10.5 mmol) of (R)-2,2'-dihydroxy-1,1'-binaphthyl, 360 mg of platinum dioxide, and 75 mℓ of acetic acid, and the mixture was stirred at 5°C for 18 hours at a hydrogen pressure of 3 atm and then at 18°C for 7 days at a hydrogen pressure of 10 atm. Ethyl acetate was added thereto, and the reaction mixture was filtered through Celite. Water was added to the filtrate for separation, and the separated organic layer was washed twice with a saturated sodium hydrogencarbonate aqueous solution, followed by separation. The organic layer was dried over magnesium sulfate, and the solvent was removed under reduced pressure to obtain 3.03 g (percent yield 98%) of the titled compound. Optical purity: ≥99 %ee.

(2) Synthesis of (R)-2,2'-bis(trifluoromethanesulfonyloxy)-5,5',6,6',7,7',8,8'-octahydro-1,1'-binaphthyl:

In a 50 mℓ four-necked flask were charged 26 mℓ of methylene chloride, 5.15 g (17.4 mmol) of (R)-5,5',6,6',7,7',8,8'-octahydro-1,1'-bi-2-naphthol, 5.58 g (52.2 mmol) of 2,6-toluidine, and 0.955 g (7.83 mmol) of 4-dimethylaminopyridine. To the solution was added 14.7 g (52.2 mmol) of trifluoromethanesulfonic acid anhydride at 0°C, followed by stirring at room temperature for 23 hours. After completion of the reaction, the solvent was removed under reduced pressure, and the residue was purified by column chromatography on silica gel using methylene chloride as a developing solvent to obtain 9.36 g (percent yield 100%) of (R)-2,2'-bis(trifluoromethanesulfonyloxy)-5,5',6,6',7,7',8,8'-octahydro-1,1'-binaphthyl.

(3) Synthesis of (R)-2-diphenylphosphinyl-2'-trifluoromethanesulfonyloxy-5,5',6,6',7,7',8,8'-octahydro-1,1'-binaphthyl:

In a 50 mℓ flask were charged 2.68 g (4.80 mmol) of (R)-2,2'-bis(trifluoromethanesulfonyloxy)-5,5',6,6',7,7',8,8'-octahydro-1,1'-binaphthyl obtained in (2) above, 1.99 g (9.04 mmol) of diphenylphosphine oxide, and 20 mℓ of dimethyl sulfoxide in an argon stream to prepare a solution. To the solution were added 108 mg (0.480 mmol) of palladium acetate, 205 mg (0.48 mmol) of 1,4-bis(diphenylphosphino)butane, 5.1 mℓ of ethyldiisopropylamine, and 33 mg (0.491 mmol) of sodium formate, and the mixture was stirred at room temperature for 20 minutes and then at 90°C for 19 hours. After cooling to room temperature, 250 mℓ of ethyl ether and 150 mℓ of water were added to the reaction mixture, followed by stirring. After separation, the organic layer separated was washed successively with four 125 mℓ portions of water, two 125 mℓ portions of 5% diluted hydrochloric acid, two 50 mℓ portions of water, 125 mℓ of a saturated

sodium hydrogencarbonate aqueous solution, and 125 mℓ of a saturated sodium chloride aqueous solution. The resulting organic layer was dried over anhydrous magnesium sulfate, and the solvent was removed under reduced pressure. The residue was purified by silica gel column chromatography using toluene/acetonitrile (3:1 by volume) as a developing solution to obtain 2.76 g (percent yield 94%) of the titled compound.

(4) Synthesis of (R)-2-diphenylphosphino-2'-hydroxy-5,5',6,6',7,7',8,8,'-octahydro-1,1'-binaphthyl:

In a 50 mℓ four-necked flask, 318.7 mg (0.664 mmol) of (R)-2-diphenylphosphinyl-2'-trifluoromethanesulfonyloxy-5,5',6,6',7,7,'8,8'-octahydro-1,1'-binaphthyl was dissolved in 22 mℓ of xylene, and 1.21 g (12 mmol) of triethylamine and 1.62 g (12 mmol) of trichlorosilane were added thereto. The reaction mixture was stirred at 120°C for 17 hours. After cooling the reaction mixture to room temperature, 4.4 mℓ of a 35% sodium hydroxide aqueous solution was carefully added thereto, followed by stirring for 2 hours. After liquid-liquid separation, the organic layer was washed with two 30 mℓ portions of a saturated sodium chloride aqueous solution and dried over anhydrous magnesium sulfate. The solvent was removed under reduced pressure. The residue was dissolved in 7 mℓ of tetrahydrofuran, and a solution of 335 mg (7.98 mmol) of lithium hydroxide in 2.4 mℓ of water was added thereto, followed by stirring at room temperature for 15 hours. To the reaction mixture were added 50 mℓ of ethyl ether and 15 mℓ of 5% diluted hydrochloric acid, followed by separation. The organic layer was washed twice with water and dried over anhydrous magnesium sulfate. The solvent was distilled off, and the residue was purified by silica gel column chromatography using hexane/ethyl acetate (5:1 by volume) as a developing solvent to obtain 105.4 mg (percent yield 51%) of the titled compound.

(5) Synthesis of (S)-1,1'-binaphthalene-2,2'-diyldioxychlorophosphine:

In a 50 mℓ flask were charged 4.94 g (17.3 mmol) of (S)-1,1'-2-binaphthol and 11.3 g (830 mmol) of phosphorus trichloride in an argon stream, and the mixture was heated at reflux for 4 hours. After cooling to room temperature, the unreacted phosphorus trichloride was removed by distillation under reduced pressure to obtain 5.56 g (percent yield 92%) of the titled compound as a colorless crystal.

(6) Synthesis of (R)-2-diphenylphosphino-5,5',6,6',7,7',8,8'-octahydro-1,1'-binaphthalen-2'-yloxy-((S)-1,1'-binaphthalene-2,2'-diyldioxy)phosphine:

In a 100 mℓ flask were charged 0.294 g (0.946 mmol) of (R)-2-diphenylphosphino-2'-hydroxy-5,5',6,6',7,7',8,8'-octahydro-1,1'-binaphthyl, 662 mg (1.89 mmol) of (S)-1,1-binaphthalene-2,2'-diyldioxychlorophosphine, and 30 mℓ of ethyl ether. To the solution was added 191 mg (1.89 mmol) of triethylamine at 0°C, followed by stirring at room temperature for 15 hours. The reaction was ceased by addition of 20 mℓ of water. After separation, the organic layer was dried over anhydrous magnesium sulfate, and the solvent was removed under reduced pressure. The residual crude product was purified by silica gel column chromatography using hexane/benzene (1:1 by volume) as a developing solution to obtain 719.4 mg (percent yield 98%) of (R)-2-diphenylphosphino-5,5',6,6',7,7',8,8'-octahydro-1,1'-binaphthalene-2'-yloxy-(S)-1,1'-binaphthalene-2,2'-diyldioxy)phosphine (hereinafter referred to as (R,S)-OcH-binaphos) as a colorless crystal.
$^{31}$P-NMR (CDCℓ$_3$) δ: -15.0 (d, J=42.7Hz), 146.8 (d, J=42.7Hz) $^{31}$P-NMR (toluene-d$_8$) δ:
50.4 (dd, $J_{p\text{-}p}$=87.0Hz, $J_{Rh\text{-}p}$=174.0Hz)
160.2 (dd, $J_{Rh\text{-}p}$=328.1Hz)

EXAMPLE 6

Synthesis of (R)-2-Diphenylphosphino-5,5',6,6',7,7',8,8'-octahydro-1,1'-binaphthalen-2'-yloxy-((S)-5,5',6,6',7,7', 8,8'-octahydro-1,1'-binaphthalene-2,2'-diyldioxy)phosphine

(1) Synthesis of (S)-5,5',6,6',7,7',8,8'-octahydro-1,1'-binaphthalene-2,2'-diyldioxychlorophosphine:

In a 50 mℓ flask were charged 5.12 g (17.3 mmol) of (S)-5,5',6,6',7,7',8,8'-octahydro-1,1'-bi-2-naphthol and 11.3 g (830 mmol) of phosphorous trichloride in an argon stream, and the mixture was refluxed for 4 hours. After cooling to room temperature, the unreacted phosphorus trichloride was removed under reduced pressure to obtain 5.7 g (percent yield 92%) of the titled compound as a colorless crystal.

(2) Synthesis of (R)-2-diphenylphosphino-5,5',6,6',7,7',8,8'-octahydro-1,1'-binaphthalen-2'-yloxy-((S)-5,5',6,6',7,7',8,8'-octahydro-1,1'-binaphthalene-2,2'-diyldioxy)phosphine (hereinafter referred to as (R,S)-$(OcH)_2$-binaphos):

(R,S)-$(OcH)_2$-binaphos was obtained in 91% yield in the same manner as in Example 1-(6), except for replacing (S)-1,1-binaphthalene-2,2'-diyldioxychlorophosphine as used in Example 1-(6) with (S)-5,5',6,6',7,7',8,8'-octahydro-1,1'-binaphthalene-2,2'-diyldioxychlorophosphine.
$^{31}$P-NMR ($CDC\ell_3$) δ: -14.7 (d, J=39.7Hz), 138.3 (d, J=39.7Hz)

EXAMPLE 7

Synthesis of (R)-2-Diphenylphosphino-1,1,'-binaphthalen-2'-yloxy((S)-1,1'-binaphthalene-2,2'-diyldioxy)phosphine

(1) Synthesis of (R)-2,2'-bis(trifluoromethanesulfonyloxy)-1,1'-binaphthyl:

9.56 g of the titled compound was obtained in 100% yield in the same manner as in Example 5-(2), except for replacing (R)-5,5',6,6',7,7',8,8'-octahydro-1,1'-bi-2-naphthol with (R)-1,1'-bi-2-naphthol.

(2) Synthesis of (R)-2-diphenylphosphinyl-2'-trifluoromethanesulfonyloxy-1,1'-binaphthyl:

2.51 g of the titled compound was obtained in 83% yield in the same manner as in Example 5-(3), except for replacing (R)-2,2'-bis(trifluoromethanesulfonyloxy)-5,5',6,6',7,7',8,8'-octahydro-1,1'-binaphthyl with (R)-2,2'-bis(trifluoromethanesulfonyloxy)-1,1'-binaphthyl.

(3) Synthesis of (R)-2-diphenylphosphino-2'-hydroxy-1,1'-binaphthyl:

153 mg of the titled compound was obtained in 51% yield in the same manner as in Example 5-(4), except for replacing (R)-2-diphenylphosphinyl-2'-hydroxy-5,5',6,6',7,7',8,8'-octahydro-1,1'-binaphthyl with (R)-2-diphenylphosphinyl-2'-hydroxy-1,1'-binaphthyl.

(4) Synthesis of (R)-2-diphenylphosphino-1,1'-binaphthalen-2'-yloxy-((S)-1,1'-binaphthalene-2,2'-diyldioxy)phosphine:

712 mg of the titled compound was obtained in 98% yield in the same manner as in Example 5-(6), except for replacing (R)-2-diphenylphosphino-2'-hydroxy-5,5',6,6',7,7',8,8'-octahydro-1,1'-binaphthyl with (R)-2-diphenylphosphino-2'-hydroxy-1,1'-binaphthyl.

EXAMPLE 8

Synthesis of (R)-2-Diphenylphosphino-1,1'-binaphthalen-2'-yloxy-((S)-10,10'-biphenanthrene-9,9'-diyldioxy) phosphine

(1) Synthesis of (S)-10,10'-biphenanthrene-9,9'-diyldioxychlorophosphine:

1.05 g of the titled compound was obtained in 90% yield in the same manner as in Example 5-(5), except for replacing (S)-1,1'-bi-2-naphthol with (S)-10,10'-bi-9-phenanthrol.

(2) Synthesis of (R)-2-diphenylphosphino-1,1'-binaphthalen-2'-yloxy-((S)-10,10'-biphenanthrene-9,9'-diyldioxy) phosphine:

726 mg of the titled compound (hereinafter referred to as (R,S)-phen-binaphos) was obtained in 95% yield in the same manner as in Example 5-(6), except for replacing (S)-1,1'-binaphthalene-2,2'-diyldioxychlorophosphine with (S)-10,10'-biphenanthrene-9,9'-diyldioxychlorophosphine.
$^{31}$P-NMR ($C_6D_6$) δ: 148.05 (d, J=21.4Hz), -13.34 (d, J=21.4Hz)

EXAMPLE 9

Preparation of Rh(acac)[(S,R)-biphemphos]

In a 20 mℓ Schlenk tube were charged 39.7 mg (0.05 mmol) of (S,R)-biphemphos of Example 1, 12.8 mg

(0.05 mmol) of $Rh(acac)_2(CO)_2$ ("acac" represents acetylacetonato, hereinafter the same), and 5 mℓ of benzene. The solution was stirred at room temperature for 5 minutes, and the solvent was removed under reduced pressure to obtain Rh(acac)[(S,R)-biphemphos] as a yellow solid in 100% yield.

EXAMPLE 10

Preparation of Rh(acac)[(R,R)-biphemphos]

Rh(acac)[(R,R)-biphemphos] was obtained in 100% yield in the same manner as in Example 9, except for replacing (S,R)-biphemphos with (R,R)-biphemphos.

EXAMPLE 11

Preparation of Rh(acac)[(R,S)-Me-biphemphos] and Rh(acac)[(S,S)-biphemphos]

The complexes Rh(acac)[(R,S)-Me-biphemphos] and Rh(acac)[(S,S)-Me-biphemphos] were prepared by the same method of Example 9.

EXAMPLE 12

Preparation of Rh(acac)[(S,R)-H-biphemphos]

Rh(acac)[(S,R)-H-biphemphos] was obtained in 100% yield in the same manner as in Example 9, except for replacing (S,R)-biphemphos with (±,R)-H-biphemphos.

EXAMPLE 13

Preparation of Rh(acac)[(R,S)-OcH-binaphos]

Rh(acac)[(R,S)-OcH-binaphos] was obtained in 100% yield in the same manner as in Example 9, except for replacing (S,R)-biphemphos with (R,S)-OcH-binaphos.

EXAMPLE 14

Preparation of Rh(acac)[(S,R)-OcH-binaphos]

Rh(acac)[(R,S)-OcH-binaphos] was obtained in 100% yield in the same manner as in Example 9, except for replacing (S,R)-biphemphos with (S,R)-OcH-binaphos.

EXAMPLE 15

Preparation of Rh(acac)[(R,S)-$(OcH)_2$-binaphos]

Rh(acac)[(R,S)-$(OcH)_2$-binaphos] was obtained in 100% yield in the same manner as in Example 9, except for replacing (S,R)-biphemphos with (R,S)-$(OcH)_2$-binaphos.

EXAMPLES 16 TO 23

Phosphine compounds shown in Tables 3 and 4 below were prepared in the same manner as in Examples 3 to 8.

EXAMPLES 24 TO 27

Phosphine compound complexes shown in Table 4 below using the phosphine compounds of Examples 20 to 23 were prepared in the same manner as in Examples 9 to 15.

TABLE 3

Example 16

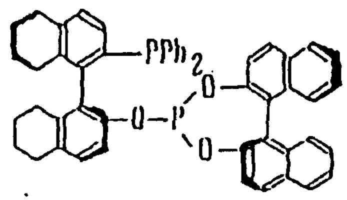

(S, R)-OcH-BINAPHOS

$^{31}$P-NMR δ :

-14.8 (d, J=40Hz)

146.8 (d, J=40Hz)

Example 17

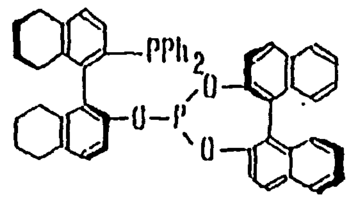

(S, S)-OcH-BINAPHOS

$^{31}$P-NMR δ :

-14.0 (br, s)

146.9 (d, J=10Hz)

Example 18

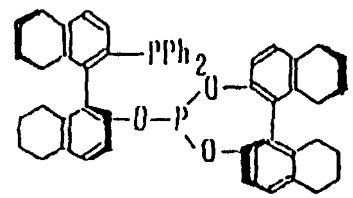

(S, R)-(OcH)$_2$-BINAPHOS

$^{31}$P-NMR δ :

-14.6 (d, J=41Hz)

139.2 (d, J=39Hz)

Example 19

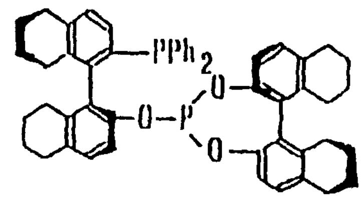

(S, S)-(OcH)$_2$-BINAPHOS

$^{31}$P-NMR δ :

-13.4 (br, s)

139.1 (d, J=8Hz)

TABLE 4

| | Examples 20 and 24 | Examples 21 and 25 | Examples 22 and 26 | Examples 23 and 27 |
|---|---|---|---|---|
| L | 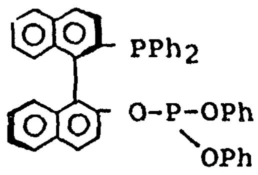 | 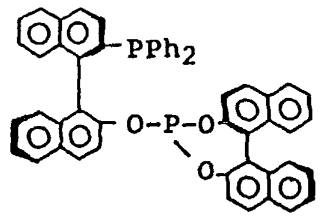 | 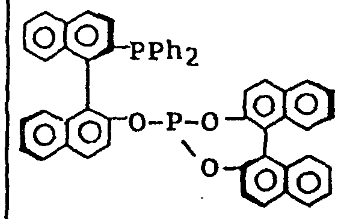 | 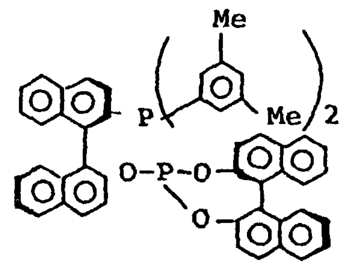 |
| Metal | (R)-Ph-binaphos<br>31P-NMR δ :<br>-10.6 (d, J=13.7Hz)<br>129.2 (d, J=13.7Hz) | (R, S)-binaphos<br>31P-NMR δ :<br>-14.6 (d, J=29.0Hz)<br>144.7 (d, J=29.0Hz) | (R, R)-(OcH)2-binaphos<br>31P-NMR δ :<br>-18.0 (d, J=9.2Hz)<br>140.5 (d, J=9.2Hz) | (R, S)-Me-binaphos<br>31P-NMR δ :<br>-12.4 (d, J=32.0Hz)<br>145.5 (d) |
| Rh | Rh(L) (acac)<br>31P-NMR δ :<br>51.1 (dd, J=82.4Hz, J=174.0Hz)<br>138.8 (dd, J=328.1Hz) | Rh(L) (acac)<br>31P-NMR δ :<br>48.3 (dd, J=83.9Hz, J=174.0Hz)<br>161.8 (dd, J=331.1Hz) | Rh(L) (acac)<br>31P-NMR δ :<br>51.9 (dd, J=80.8Hz, J=178.4Hz)<br>161.8 (dd, J=335.1Hz) | Rh(L) (acac)<br>31P-NMR δ :<br>48.8 (dd, J=82.4Hz, J=172.4Hz)<br>160.9 (dd, J=332.6Hz) |

EXAMPLE 28

Preparation of (S)-(+)-2-Phenylpropanal

In a 50 mℓ autoclave were charged 38.7 mg (0.0488 mmol) of (S,R)-biphemphos, 3.1 mg (0.012 mmol) of Rh (acac)$(CO)_2$, and a solution of 1250 mg (12.0 mmol) of styrene in 2.4 mℓ of benzene, and hydrogen and carbon monoxide were fed thereto each to a partial pressure of 50 atm. The mixture was stirred at 60°C for 42 hours. A conversion of the starting olefin and an aldehyde production ratio were determined by $^1$H-NMR (internal standard: diphenylmethane) analysis, and an optical purity (enantiomer excess) was determined by GLC analysis after conversion of the resulting optically active aldehyde into the corresponding carboxylic acid by Jones oxidation. As a result, the conversion of styrene was 100%, and the 2-phenylpropanal/3-phenylpropanal production ratio was 9:1. The 2-phenylpropanal produced had an optical purity of (S)-(+)-2-phenylpropanal of 94 %ee.

EXAMPLES 29 TO 36

Hydroformylation of olefins shown in Table 5 below was carried out in the same manner as in Example 24, except for using the phosphine compound shown in Table 5 and changing the reaction conditions as shown in Table 5. The reaction results obtained are shown in Table 5.

## TABLE 5

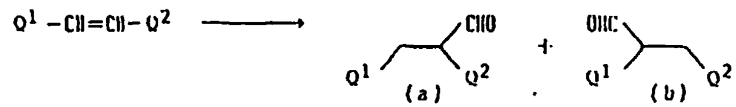

(a) (b)

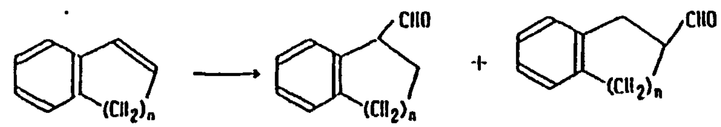

n=1,2 (c) (d)

| Example No. | Substrate | Phosphine Compound | Reaction Conditions Temp. (°C) | Time (hr) | S/C* | Conversion (%) | (a)/(b) or (c)/(d) | Optical Purity of (a) or (c) (%ee) | Absolute Configuration or Specific Rotation |
|---|---|---|---|---|---|---|---|---|---|
| 29 | $Q^1$=H, $Q^2$=$C_6H_5$ | (R,R)-biphemphos | 60 | 40 | 1000 | 95 | 92/8 | 16 | R-(-) |
| 30 | $Q^1$=H, $Q^2$=$C_6H_5$ | (S,R)-H-biphemphos + (R,R)-H-biphemphos | 60 | 40 | 1000 | 98 | 89/11 | 69 | S-(+) |
| 31 | $Q^1$=H, $Q^2$=$OCOCH_3$ | (S,R)-biphemphos | 60 | 40 | 1000 | 65 | 85/15 | 90 | R-(+) |
| 32 | $Q^1$=H, $Q^2$=$OCOCH_3$ | (R,S)-Me-biphemphos | 60 | 40 | 1000 | 83 | 89/11 | 91 | S-(-) |
| 33 | $Q^1$=H, $Q^2$=n-$C_4H_9$ | (S,R)-biphemphos | 30 | 40 | 1000 | 51 | 23/77 | 85 | S-(+) |
| 34 | $Q^1$=$Q^2$=$CH_3$ | (S,R)-biphemphos | 60 | 40 | 3500 | -** | - | 85 | R-(-) |
| 35 | indene (n=1) | (S,R)-biphemphos | 60 | 20 | 200 | 62 | 92/8 | 88 | (+) |
| 36 | 1,2-dihydronaphthalene (n=2) | (R,S)-biphemphos | 60 | 12 | 500 | 74 | 95/5 | 96 | (-) |

Note: * S/C is a substrate/rhodium compound ratio.
** The conversion of 2-butene was measured per hour because of high volatility of 2-butene. As a result, the conversion was 3.7%.

EXAMPLE 37

Preparation of 2-Acetoxypropanal

In a 50 mℓ autoclave were charged 761 mg (8.84 mmol) of vinyl acetate, 8.6 mg (0.00884 mmol) of Rh(acac) ((R,S)-OcH-binaphos), and 8.3 mℓ of benzene, and 50 atm of hydrogen and 50 atm of carbon monoxide were fed thereto. The mixture was stirred at 60°C for 44 hours. The conversion of vinyl acetate was 72%. The product was a mixture comprising 88.6% of 2-acetoxypropanal and 11.4% of 3-acetoxypropanal. The optical purity of the 2-acetoxypropanal was 90 %ee.

EXAMPLES 38 TO 42

Hydroformylation of styrene or vinyl acetate was carried out in the same manner as in Example 28 or 37, except for using the catalyst or catalyst system shown in Table 6 below and changing the reaction conditions as shown in Table 6. The reaction results obtained are shown in Table 6.

TABLE 6

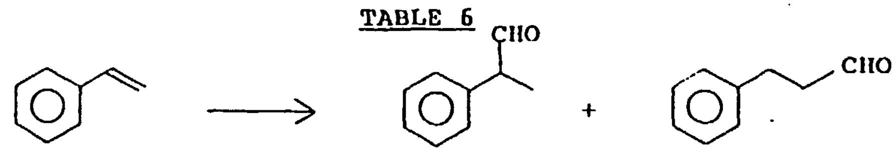

(e) (f)

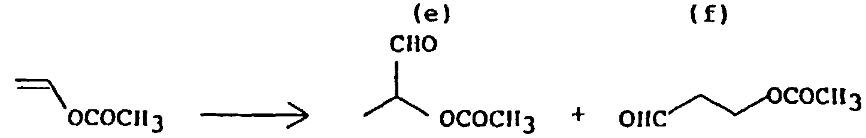

(g) (h)

| Example No. | Substrate | Catalyst or Catalyst System | Reaction Conditions Temp. (°C) | Time (hr) | S/C* | Conversion (%) | (e)/(f) or (g)/(h) | Optical Purity of (e) or (g) (%ee) | Absolute Configuration or Specific Rotation |
|---|---|---|---|---|---|---|---|---|---|
| 38 | styrene | $Rh(CO)_2(acac)$ + (S,R)-binamphos | 60 | 43 | 2056 | 100 | 88/12 | 94 | S-(+) |
| 39 | styrene | Rh((S,R)-OcH-binaphos)(acac) | 60 | 100 | 226 | 100 | 93/7 | 93 | |
| 40 | styrene | $Rh((R,S)-(OcH)_2$-binaphos)(acac) | 60 | 100 | 226 | 84 | 92/8 | 89 | |
| 41 | vinyl acetate | $Rh(CO)_2(acac)$ + (R,S)-binamphos | 60 | 112 | 400 | 98 | 90/10 | 90 | S-(-) |
| 42 | vinyl acetate | Rh((R,R)-binaphos)(acac) | 60 | 37 | 400 | 46 | 92/8 | 73 | S-(-) |

Note: * S/C is a substrate/rhodium compound ratio.

EXAMPLE 43

Preparation of (R)-2-[(3S,4R)-3-[(R)-1-t-butyldimethylsilyloxyethyl]-2-oxoazetidine-4-yl]propanal

In a 100 ml autoclave were charged 9.3 mg (0.01 mmol) of Rh(acac)(R,S)(R,S)-binaphos,8 mg (0.01 mmol) of (R,S)-binaphos, and 510 mg (2 mmol) of (1'R,3S,4R)-3-[1'-[(t-butyldimethylsilyl)oxy]ethyl]-4-vinylazetidine-2-one. After throughly purging the autoclave with nitrogen, 5 ml of toluene was added thereto.

Carbon monoxide was introduced into the autoclave under a pressure of 50 atm, and hydrogen was then introduced to a pressure of 100 atm. The mixture was vigorously stirred while heating at 60°C in an oil bath for 48 hours. After cooling to room temperature, the excess carbon monoxide and hydrogen were released, and toluene was distilled off under reduced pressure. Purification of the residue by silica gel column chromatography afforded 513 mg (90%) of a 94:6 mixture of (R)-2-[(3S,4R)-3-[(R)-1-t-butyldimethylsilyloxyethyl]-2-oxoazetidine-4-yl]propanal.

The 2(R)/2(S) ratio was decided from the integrated ratio and aldehyde proton by $^1$H-NMR. Further, the product as obtained was subjected to Jones oxidation to the corresponding mixed carboxylic acid. The resulting mixture was analyzed by high-performance liquid chromatography (Inertsil ODS-2; eluent: acetonitrile/water = 6:4; pH = 2.3 ($H_3PO_4$)), and the results obtained were compared with those of a standard sample.

2(R)-Isomer:

$^1$H-NMR ($CDC\ell_3$) $\delta$:

0.07 (s,3H), 0.08 (s,3H), 0.88 (s,9H), 1.22 (d, J=7.3Hz, 3H), 1.24 (d, J=6.3Hz, 3H), 2.68 (m,1H), 3.94 (dd, J=5.4, 2.4Hz, 1H), 4.20 (m, 1H), 5.98 (s, 1H), 9.81 (J=1.01Hz)

EXAMPLES 44 TO 50

A (2R/2S)-mixed formylated compound was obtained in the same method as in Example 43 except for using the catalyst or precursor shown in Table 7 below.

TABLE 7

| Example No. | Catalyst | 2R/2S | Yield (%) |
|---|---|---|---|
| 44 | $[Rh(COD)C\ell]_2$ + (R,S)-binaphos | 94/6 | 89 |
| 45 | Rh(acac)((R,S)-OcH-binaphos) | 94/6 | 70 |
| 46 | $[Rh(COD)C\ell]_2$ + (R,S)-$(OcH)_2$-binaphos | 65/35 | 65 |
| 47 | Rh(acac)((R,S)-bin-OcH-binaphos)* | 52/48 | 70 |
| 48 | $Rh(CO)_2(acac)$ + (R,S)-biphemphos | 93/7 | 75 |
| 49 | $Rh(CO)_2(acac)$ + (R,S)-phen-binaphos | 95/5 | 70 |
| 50 | $Rh(CO)_2(acac)$ + (R,S)-$Me_2$-binaphos** | 92/8 | 40 |

*: (R)-2-diphenylphosphino-1,1'-binaphthalene-2'-yloxy-((S)-5,5',6,6',7,7',8,8'-octahydro-1,1'-binaphthalene-2,2'-diyldioxy)phosphine

**: (R)-2-diphenylphosphino-1,1'-binaphthalene-2'-yloxy-((S)-3,3'-dimethyl-1,1'-binaphthalene-2,2'-diyldioxy)phosphine

EXAMPLES 51 TO 53

A (2R/2S)-mixed formylated compound was obtained in the same method as in Example 43, except for using the solvent shown in Table 8 below.

TABLE 8

| Example No. | Solvent | 2R/2S | Yield (%) |
|---|---|---|---|
| 51 | benzene | 94/6 | 90 |
| 52 | dimethoxyethane | 84/16 | 62 |
| 53 | ethyl acetate | 78/22 | 89 |

According to the present invention, optically active aldehydes can be obtained with high regio and enantio selectivities by hydroformylation of olefins in the presence of a combination of the specific phosphine compound according to the present invention and a transition metal compound or in the presence of a complex prepared from the phosphine compound and the transition metal compound.

## Claims

**1.** A 4-[(R)-1'-formylethyl]azetidin-2-one derivative represented by formula (VII):

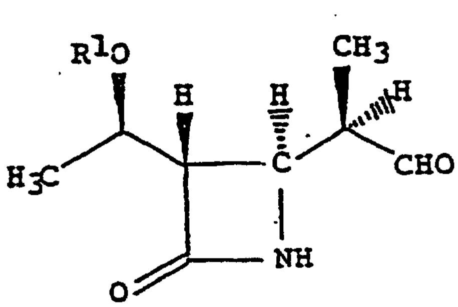

(VII)

wherein R1 represents a hydrogen atom or a hydroxyl-protecting group.

**2.** Use of a derivative as claimed in Claim 1 in the preparation of carbapenem antibiotics.

**3.** A process for preparing a 4-[(R)-1'-formylethyl]azetidin-2-one derivative of formula (VII) comprising hydroformylation of 4-vinylazetidin-2-one derivatives in the presence of a phosphine complex composed of a transition metal and a compound represented by formula (I)

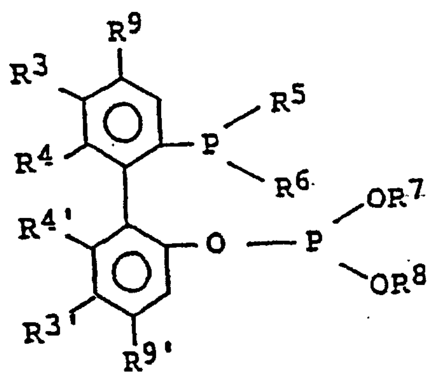

(I)

wherein $R^4$ and $R^{4'}$, which may be the same or different, each represent a hydrogen atom, a lower alkyl group or a lower alkoxy group; $R^3$, $R^{3'}$, $R^9$, and $R^{9'}$, which may be the same or different, each represent a hydrogen atom, a lower alkyl group, a lower alkoxy group or a halogen atom; or a pair of $R^3$ and $R^4$ or a pair of $R^{3'}$ and $R^{4'}$ may form a ring; $R^5$ and $R^6$, which may be the same or different, each, represent a phenyl group or a phenyl group substituted with a lower alkyl group, a halogen atom or a lower alkoxy group; and $R^7$ and $R^8$ which may be the same or different, each represent a phenyl group or a phenyl group substituted with a lower alkyl group, a lower alkoxy group or a halogen atom; or $R^7$ and $R^8$ may be taken together to form a divalent hydrocarbon group.

## Patentansprüche

**1.** 4-[(R)-1'-Formylethyl]azetidin-2-on-Derivat, das durch die Formel (VII) wiedergegeben wird:

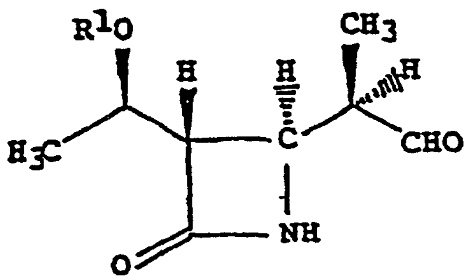

(VII)

worin $R^1$ ein Wasserstoffatom oder eine Hydroxyl-Schutzgruppe bedeutet.

**2.** Verwendung eines Derivats, wie in Anspruch 1 beansprucht, bei der Herstellung von Carbapenem-Antibiotika.

**3.** Verfahren zum Herstellen eines 4-[(R)-1'-Formylethyl]azetidin-2-on-Derivats der Formel (VII), umfassend die Hydroformylierung von 4-Vinylazetidin-2-on-Derivaten in Gegenwart eines Phosphinkomplexes, der sich aus einem Übergangsmetall und einer Verbindung zusammensetzt, die durch die Formel (I) wiedergegeben wird:

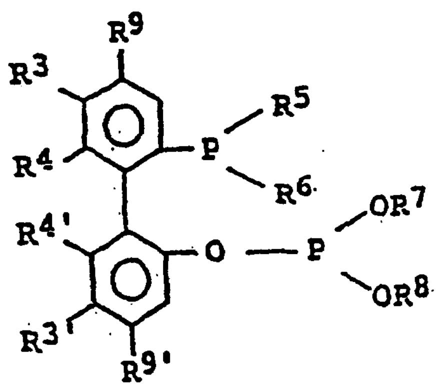

(I)

worin $R^4$ und $R^{4'}$, welche gleich oder verschieden sein können, jeweils ein Wasserstoffatom, eine niedere Alkylgruppe oder eine niedere Alkoxygruppe bedeuten, $R^3$, $R^{3'}$, $R^9$ und $R^{9'}$, welche gleich oder verschieden sein können, jeweils ein Wasserstoffatom, eine niedere Alkylgruppe, eine niedere Alkoxygruppe oder ein Halogenatom bedeuten; oder ein Paar von $R^3$ und $R^4$ oder ein Paar von $R^{3'}$ und $R^{4'}$ einen Ring bilden kann; $R^5$ und $R^6$, welche gleich oder verschieden sein können, jeweils eine Phenylgruppe oder eine Phenylgruppe, die mit einer niederen Alkylgruppe, einem Halogenatom oder einer niederen Alkoxygruppe substituiert ist, bedeuten; und $R^7$ und $R^8$, welche gleich oder verschieden sein können, jeweils eine Phenylgruppe oder eine Phenylgruppe, die mit einer niederen Alkylgruppe, einer niederen Alkoxygruppe oder einem Halogenatom substituiert ist, bedeuten; oder $R^7$ und $R^8$ zusammengenommen eine zweiwertige Kohlenwasserstoffgruppe bilden können.

## Revendications

**1.** Dérivé de 4-[(R)-1'-formyléthyl]azétidin-2-one représenté par la formule (VII) :

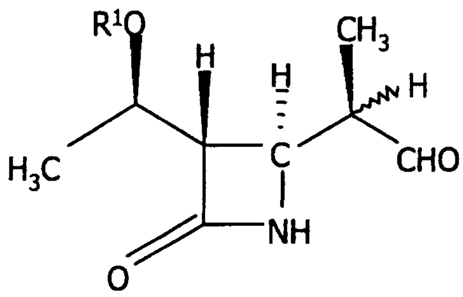

(VII)

où $R^1$ représente un atome d'hydrogène ou un groupe protecteur d'hydroxyle.

**2.** Utilisation d'un dérivé selon la revendication 1 dans la préparation d'antibiotiques de type carbapénème.

**3.** Procédé pour préparer un dérivé de 4-[(R-1'-formyléthyi]azétidin-2-one de formule (VII) comprenant l'hydroformylation de dérivés de la 4-vinylazétidin-2-one en présence d'un complexe de phosphine composé d'un métal de transition et d'un composé représenté par la formule (I)

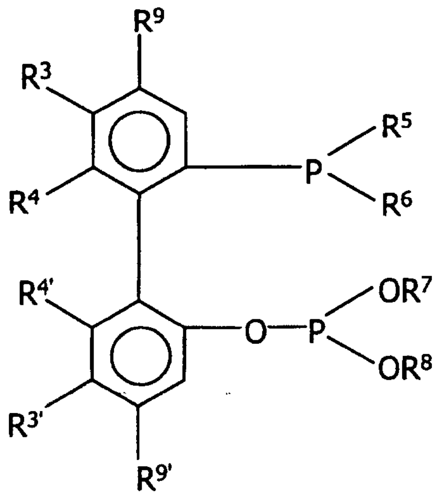

(I)

où $R^4$ et $R^{4'}$, qui peuvent être identiques ou différents, représentent chacun un atome d'hydrogène, un groupe alkyle inférieur ou un groupe alcoxy inférieur ; $R^3$, $R^{3'}$, $R^9$ et $R^{9'}$, qui peuvent être identiques ou différents, représentent chacun un atome d'hydrogène, un groupe alkyle inférieur, un groupe alcoxy inférieur ou un atome d'halogène ; ou une paire de $R^3$ et $R^4$ ou une paire de $R^{3'}$ et $R^{4'}$ peuvent former un cycle ; $R^5$ et $R^6$, qui peuvent être identiques ou différents, représentent chacun un groupe phényle ou un groupe phényle substitué par un groupe alkyle inférieur, un atome d'halogène ou un groupe alcoxy inférieur ; et $R^7$ et $R^8$, qui peuvent être identiques ou différents, représentent chacun un groupe phényle ou un groupe phényle substitué par un groupe alkyle inférieur, un groupe alcoxy inférieur ou un atome d'halogène ; ou bien $R^7$ et $R^8$ peuvent être combinés pour former un groupe hydrocarboné divalent.